# EUROPEAN PATENT SPECIFICATION

(11) **EP 2 326 357 B1**
(45) Date of publication and mention of the grant of the patent: **19.09.2012**
(21) Application number: 09792768.5
(22) Date of filing: 21.09.2009
(51) Int. Cl.: A61L 27/48, A61L 27/50

(54) **COMPOSITION AND METHOD FOR TREATING TISSUE DEFECTS**
ZUSAMMENSETZUNG UND VERFAHREN ZUR BEHANDLUNG VON GEWEBEDEFEKTEN
COMPOSITION ET PROCÉDÉ DE TRAITEMENT DE DÉFAUTS DE TISSU

(30) Priority: 26.09.2008 US 238991
(43) Date of publication of application: 01.06.2011
(73) Proprietor: Ethicon, Inc., Somerville, NJ 08876 (US)
(72) Inventor: ROUSSEAU, Robert, A., Ottsville, PA 18942 (US); WEADOCK, Kevin, S., Hillsborough, NJ 08844 (US); JACOBS, John, R., Easton, PA 18040 (US)
(74) Representative: James, Anthony Christopher W.P.
(86) International application number: PCT/US2009/057661
(87) International publication number: WO 2010/036604

(56) References cited:
- US-A1- 2006 093 644
- US-B1- 6 432 437
- US-B1- 6 716 251

## Description

### FIELD OF THE INVENTION

The present invention relates to kits for providing devices and methods for preparing compositions for treating tissue defects, and more particularly, to such devices and methods including microparticles or fibers of a nonresorbable polymer within a matrix material comprised of a surfactant and a resorbable polymer.

### BACKGROUND OF THE INVENTION

As a result of aging, trauma, disease, or congenital malformations, the mammalian body is frequently affected by a variety of tissue defects. These tissue defects can be in the form of a hernia, aneurysm, scar, seroma or in the form of excessive tissue laxity in the skin, soft palate or pharyngeal wall. A hernia is a well known type of defect in the abdominal wall, in which the inside layers of the abdominal muscle have weakened resulting in a bulge or tear. In the same way that an inner tube pushes through a damaged tire, the inner lining of the abdomen pushes through the weakened area of the abdominal wall to form a small bubble or balloon-like sac. When a loop of intestine or abdominal tissue pushes into the sac, severe pain and other potentially serious complications can result. Hernias most commonly occur in the groin (inguinal hernia), around the navel (umbilical hernia), and near the site of a previous surgical operation (incisional hemia).

Approximately 800,000 hernia repair operations are performed annually in the United States, many of which are performed by the conventional "open" method, which has been the gold standard for over 100 years. Due to the larger size of the incision, however, open hernia repair is generally painful with a relatively long recovery period. Minimally invasive (laparoscopic) repair has been developed over the past decade, and is a relatively new surgical technique to fix tears in the abdominal wall (muscle) using small incisions, a patch (mesh), and special cameras to view inside the body. It frequently offers a more rapid recovery for the patient, less postoperative pain, and a quicker return to work and normal activities. Nonetheless, the laparoscopic procedure is a surgical procedure requiring anesthesia, several incisions, expensive surgical instruments, and hospitalization. In some patients, the presence of a mesh may increase the chance of infection, seroma, and post-operative pain. In addition, poorly positioned mesh may also result in hernia recurrence.

Other surgical approaches to repairing these defects include suturing defects closed, using meshes to support defects, or injecting material to augment those tissue defects that have noticeable voids. With regard to injectable materials, injectable fillers have been used to reduce the appearance of wrinkles and as bulking agents for other applications such as to address urinary incontinence. These fillers typically consist of entirely resorbable materials such as collagen or hyaluronic acid that necessitate periodic injections to maintain the desired result. Non-resorbable polymers, such as ethylene vinyl alcohol copolymer (EVOH) in a solvent carrier such as dimethyl sulfoxide (DMSO) have also been used as bulking agents in the treatment of gastroesophageal reflux disease(GERD). EVOH is prepared by the polymerization of ethylene and vinyl acetate, followed by hydrolysis.

Challenges associated with using injectable fillers to repair tissue defects include the need to maintain the filler at the desired location (avoid migration), while at the same time, at least for resorbable fillers, to stimulate tissue generation and growth to incorporate rather than encapsulate the injected material. The desired injectable filler should be suitable for delivery in a minimally invasive manner that so that the tissue defect is restored back to a functional state and proper anatomical configuration, and so that the filler conforms to the tissue defect site and result in a decreased risk of seroma, post-operative pain, or infection. It would also be desirable for such an injectable material to be usable to treat a variety of tissue defects in a mammal, including, but not limited to, aortic dissection, pelvic floor prolapse, obstructive sleep apnea, gastroesophogeal reflux disease (GERD), fecal incontinence, urinary incontinence, bronchoscopic lung volume reduction, or as a bulking agent for the correction of moderate to severe facial wrinkles and folds, such as nasolabial folds, facial fat loss (lipoatrophy), vocal cord insufficiency, craniofacial augmentation and radiographic tissue marking.

US 2006/0093644 A1 discloses medical implants that comprise microspheres and autologous carrier materials such as tissues, cells and fluids to reduce the likelihood of viral infection and eliminate adverse reactions from the patient's body to foreign materials.

### SUMMARY OF THE INVENTION

According to a first aspect of the present invention there is provided a kit, as set out in claim 1.

The kit in use provides the device.

Preferred features of the kit of the present invention are set out in claims 2 to 11.

A method is also provided for preparing a composition for treating tissue defects in mammals, where the method includes the steps of providing first and second elements capable of polymerizing to form a resorbable polymer when combined, providing a surfactant, providing non-resorbable polymer microparticles, and combining the first and second elements with the surfactant and microparticles to form a resorbable polymer matrix having the non-resorbable microparticles dispersed therein, wherein the first and second elements are thrombin and fibrinogen, or sodium alginate and calcium chloride.

The combining step can occur in situ to treat the tissue defect, and may further include, following the combining step, implanting the complex within a patient to treat the tissue defect.

The tissue defect may be selected from the group consisting of hernia, acne, wrinkles, emphysematous bullae, seroma, anal sphincter deficiency, and urinary sphincter deficiency.

The combining step can occur in the uvula, soft palate, or pharyngeal wall.

These and other features and advantages of the present invention will become apparent from the following more detailed description, when taken in conjunction with the accompanying drawings which illustrate, by way of example, the principles of the invention.

### BRIEF DESCRIPTION OF THE FIGURES

**FIGURE 1** is an illustration of an exemplary hernia defect.
**FIGURE 2** is an illustration of the hernia defect of Fig. 1 following treatment according to the present invention.
**FIGURE 3** is an image of a composition according to the present invention.
**FIGURE 4** illustrates an exemplary composition according to the present invention when used for treating acne scars.

### DETAILED DESCRIPTION

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. For example, although the present invention is described in particular in relation to hernia repair and sleep apnea, it is to be understood that it can be readily adapted for treatment or repair of other tissue defects as well.

The injectable nature of the composition of the present invention enables non-invasive, percutaneous delivery through a needle, syringe, injection syringe, cannula, trocar, or any other suitable applicator. Small diameter needles (e.g., 27G) can be used, which is a valuable attribute when treating facial wrinkles and folds, sleep apnea, nasolabial folds, facial fat loss (lipoatrophy), vocal cord insufficiency, craniofacial augmentation and radiographic tissue marking. As will be described in greater detail below, the composition is comprised of microparticles or microfibers of a nonresorbable polymer within a matrix of a resorbable material. The resorbable nature of the matrix enables tissue ingrowth over time, while the non-resorbable nature of the microparticles or microfibers allows residual material to be left behind to ensure longevity of the repair, but also serves to exacerbate the tissue response, causing inflammation or irritation that provides continuous stimulation of collagen production by the native tissue. The microparticles also serve as a malleable scaffold for native tissue ingrowth. As opposed to a solid implant, such malleability allows the native tissue to control the architecture of the healing process. The composition of the present invention further includes a surface active agent or surfactant that serves to keep the non-resorbable material sufficiently well dispersed within the composition. The uniform distribution enables the composition to be delivered to the site of a tissue defect in a predictable manner, i.e., it will not settle in one area as is noted when a surfactant is not used.

The compositions and methods described herein may be used to treat various tissue defects, and preferably is comprised of a resorbable matrix that contains a surface active agent and microparticles of a non-resorbable material. As stated, the microparticles serve to stimulate tissue to form within the defect so that as the resorbable matrix resorbs, native tissue ingrowth replaces it. The surface active agent serves to keep the polymeric microparticles dispersed in a substantially uniform manner. A surface active agent (surfactant) possesses approximately an equal ratio between the polar and nonpolar portions of each molecule. When placed in a polymer-water system, the polar groups are attracted to or orient toward the water, and the nonpolar groups are oriented toward the hydrophobic polymer. The surfactant molecule lowers the interfacial tension between the polymer and water phases. Surfactants are classified as cationic (Zephiran™), anionic (Aerosol OT™) and nonionic (Tween™) based on the type of polar group on the surfactant. A preferred surfactant used in the preferred embodiment of the present invention is the nonionic Tween-60. The microparticles are preferably non-resorbable polymer microparticles, but may also be materials such as ceramic, resorbable silicon or metal. The particles may be spherical, fibrous, stellate, or any suitable shape or configurations, and preferably have dimensions of 1-1000 microns. Once implanted, the matrix containing the particles cures to form a compliant film or body that traps the uniformly suspended microparticles. In the preferred embodiment, the matrix is adherent to surrounding tissue in order to avoid migration. The microparticles also serve as scaffolds for this newly forming tissue.

The compositions described herein may be delivered percutaneously via a needle, small diameter catheter or cannula or the like. As a result, there is less pain, reduced chance of trauma to surrounding tissues (such as nerves and blood vessels) and a shorter recovery time. Since the bulk of the material resorbs away and is replaced with native tissue, there is less chance of the device serving as a nidus for infection. Other adverse responses such as seroma may also be reduced since the composition reduces the volume of free tissue space.

The matrix of the present invention is preferably comprised of resorbable polymers such as poly(amino acids); proteins; and poly(peptides); poly(esters) such as poly(lactic acid), poly(glycolic acid), poly(lactic-co-glycolic acid), and poly(caprolactone); poly(anhydrides); poly(orthoesters); poly(carbonates); and chemical derivatives thereof (substitutions, additions of chemical groups, for example, alkyl, alkylene, hydroxylations, oxidations, and other modifications routinely made by those skilled in the art), copolymers and mixtures thereof.

Particles useful for the microparticles of the present invention are preferably selected from nonresorbable polymers such as polypropylene, polyethylene terephthalate, polytetrafluoroethylene, poly(ethers) such as poly(ethylene oxide), poly(ethylene glycol), and poly(tetramethylene oxide); vinyl polymers-poly(acrylates) and poly(methacrylates) such as methyl, ethyl, other alkyl, hydroxyethyl methacrylate, acrylic and methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; poly(siloxanes); and any chemical derivatives thereof (substitutions, additions of chemical groups, for example, alkyl, alkylene, hydroxylations, oxidations, and-other modifications routinely made by those skilled in the art), copolymers and mixtures thereof. Silicone or pyrolytic carbon microparticles may also be suitable for the present invention.

According to one embodiment, a biological sealant such as fibrin glue may be used as the resorbable matrix. Fibrin sealants are used as hemostats, sealants, tissue adhesives, and as a matrix for substances/cells in a number of surgical and tissue engineering procedures. Attractive characteristics of fibrin glue are high tensile strength, adhesive strength, biocompatibility, and resorption. Polypropylene microparticles can be mixed with a buffered solution of thrombin and surfactant (i.e., Tween-60) and then placed within one chamber of a dual chamber syringe or the like. The thrombin is preferably dissolved at a concentration within the range of 200 - 2000 IU/ml in a suitable buffer such as physiologic saline or phosphate buffered saline. A solution of fibrinogen in a suitable buffer such as phosphate buffered saline or physiologic saline is placed in the other chamber. The concentration of fibrinogen is preferably within the range of 10-100 mg/ ml. The fibrinogen solution may also contain other clotting factors such as von Willebrand factor or Factor XIII. If desired, the microparticles and surfactant can also be mixed with the fibrinogen solution. After performing any necessary dissection in or around the tissue defect, the two solutions can then be injected simultaneously into the site, e.g., a hernia. If necessary, another needle or cannula can be placed at the site to vent any excess fluid or air that may have been formed in the area near the tissue defect.

In another embodiment, sodium alginate is used as a matrix material. In this embodiment, microparticles of polymer such as polyethylene or polypropylene can be added to a solution of sodium alginate and surfactant. A uniformly distributed dispersion of microparticles is thus obtained. During implantation, a dual chamber syringe can then be used to mix the dispersion with a solution of calcium chloride; thereby causing the sodium alginate to crosslink and trap the microparticles. Simultaneous injection of the dispersion of microparticles and solution of calcium chloride will allow for treatment of an anatomical defect such as a hernia space or acne scar, for example.

Therapeutic agents can also be added to the kit to modulate the healing response. The incorporation of hydrophilic moieties such as gelatin or other hydrogels to the device may allow a range of therapeutic agents with varying solubilities to be delivered. Suitable therapeutic agents include vinca alkaloids, paclitaxel, antibiotics, enzymes; antiplatelet agents such as GPIIa/IIIb inhibitors and vitronectin receptor antagonists; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; anti-inflammatory agents such as adrenocortical steroids (cortisol, cortisone, fludrocortisone, prednisone, prednisolone, 6α-methylprednisolone, triamcinolone, betamethasone, and dexamethasone), non-steroidal agents such as ibuprofen, aspirin, acetominophen; indomethacin; angiogenic agents such as vascular endothelial growth factor (VEGF), fibroblast growth factor (FGF); and protease inhibitors. Lidocaine and other anesthetics can also be added to decrease pain at the injection site. Antimicrobial agents or antibiotics may also be added to reduce the likelihood of local infection.

Other therapeutic agents that can be added to the kit include growth factors and inflammatory cytokines involved in angiogenesis, fibroblast migration, fibroblast proliferation, ECM synthesis and tissue remodeling, such as epidermal growth factor (EGF) family, transforming growth factor-α (TGF-α), transforming growth factor-β (TGF-9-1, TGF-9-2, TGF-9-3, platelet-derived growth factor (PDGF), fibroblast growth factor (acidic--aFGF; and basic--bFGF), fibroblast stimulating factor-1, activins, vascular endothelial growth factor (including VEGF-2, VEGF-3, VEGF-A, VEGF-B, VEGF-C, placental growth factor--PIGF), angiopoietins, insulin-like growth factors (IGF), hepatocyte growth factor (HGF), connective tissue growth factor (CTGF), myeloid colony-stimulating factors (CSFs), monocyte chemotactic protein, granulocyte-macrophage colony-stimulating factors (GM-CSF), granulocyte colony-stimulating factor (G-CSF), macrophage colony-stimulating factor (M-CSF), erythropoietin, interleukins (particularly IL-1, IL-8, and IL-6), tumor necrosis factor-α (TNF9), nerve growth factor (NGF), interferon-α, interferon-β, histamine, endothelin-1, angiotensin II, growth hormone (GH), and synthetic peptides, analogues or derivatives of these factors.

Furthermore, addition of anti-fibrinolytic agents such as tranexamic acid to the composition may also be desired. Tranexamic acid (commonly marketed as Cyklokapron in the U.S. and as Transamin in Asia) is often prescribed for excessive bleeding. It is an antifibrinolytic that competitively inhibits the activation of plasminogen to plasmin, a molecule responsible for the degradation of fibrin. It has roughly eight times the antifibrinolytic activity of an older analogue, ε-aminoacaproic acid. These agents will prolong the resorption rate of the fibrin glue in vivo and may also improve the short-term mechanical properties of the implant once implanted.

Other agents such as irritants may be added to the kit to aid in stimulating tissue growth and adhesion formation during the healing process. Such agents may include silk, talc, talcum powder, copper, metallic beryllium, wool, quartz dust, silica, crystalline silicates, polylysine, polyurethanes, poly(ethylene terephthalate), polytetrafluoroethylene (PTFE), poly(alkylcyanoacrylates), and poly(ethylene-co-vinylacetate); vinyl chloride and polymers of vinyl chloride; and peptides with high lysine content. Other examples include inflammatory microcrystals such as crystalline silicates; bromocriptine, methylsergide, methotrexate, chitosan, N-carboxybutyl chitosan, carbon tetrachloride, thioacetamide, fibrosin, ethanol, and bleomycin. Radiopaque agents such as micronized tantalum powder can also be added to the composition to aid in imaging that may be desired either during or after implantation.

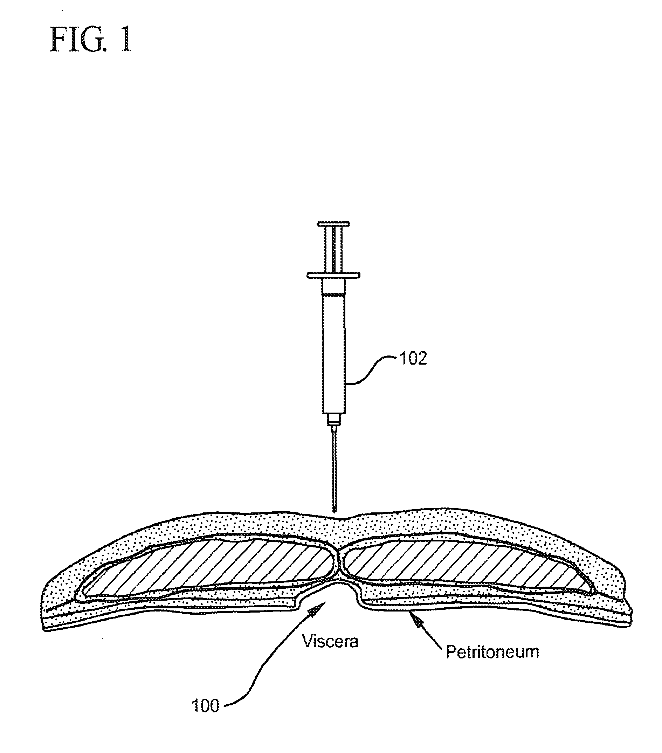

Referring now to particular applications, when used to treat a hernia, the utility of the resorbable matrix component of the device is that it is initially flowable but with sufficient viscosity to reduce the hernia sac. In addition, it may cure in place after the sac is reduced as described in more detail in the examples below. Furthermore, the gradual resorption of the material allows for native tissue ingrowth, the native tissue ingrowth being more compliant than conventional mesh products. In addition, the likelihood of infection after implantation is reduced, since the size of the nidus for bacterial colonization is reduced. The method of delivery involves locating the hernia defect, inserting the syringe, catheter, or cannula to a location proximate the tissue defect 100 as illustrated in Fig. 1, and delivering the device until the defect has been treated. Means for accelerating the curing of the matrix in situ may optionally be part of the delivery system. Such means may be a light source or addition of a chemical initiator or crosslinking agent for polymerization.

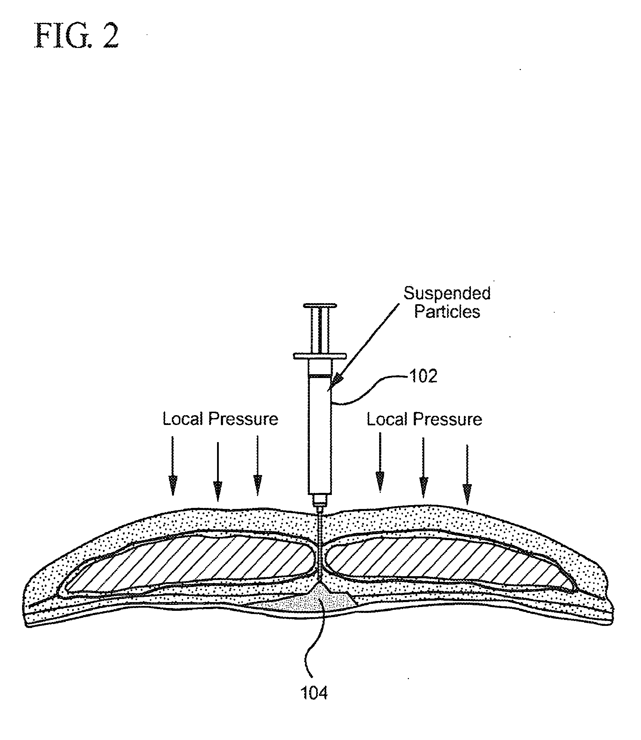

When delivering the composition to the tissue defect as shown in Fig. 2, a delivery device 102 such as a syringe, catheter, or cannula capable of containing a sufficient amount of the composition, e.g., approximately 0.1 - 100 ml, to treat the patient in one injection may be used. The delivery device preferably has at least one opening to allow the device to enter the implant site. The method can also comprise the use of a containment means such as a ring or plate to prevent the composition from migrating too far from the site of the tissue defect. This unwanted effect could cause undue dissection of the subcutaneous space. The site where the composition is delivered to may optionally be vented with another needle or cannula so that air or other material such as bodily fluids can be removed. This can help prevent excessive or unwanted dissection. Fluoroscopic guidance can also be considered as an adjunct means for accurately injecting the composition into the desired location. In this case, the presence of a radiopaque substance such as micronized tantalum powder in the composition would be advantageous.

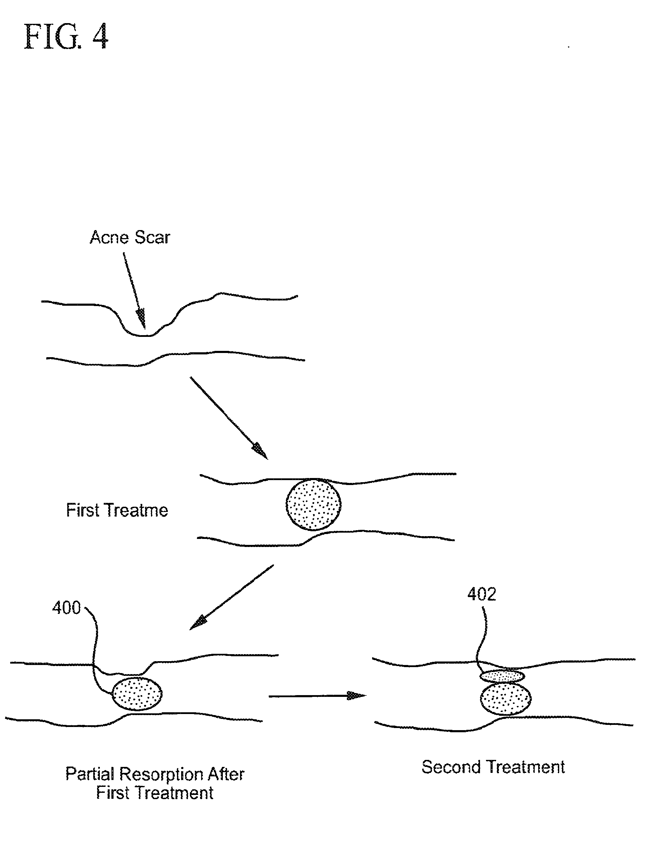

Due to its flowable properties and utility in promoting native tissue growth about a defect, the composition can also have applications in other surgical needs. These include aortic dissection, bronchoscopic lung volume reduction, pelvic floor prolapse, and as a bulking agent for disorders such as severe acne scars, obstructive sleep apnea, gastroesophageal reflux disease (GERD), fecal or urinary incontinence, and seroma prevention. For example, when treating facial wrinkles or the like as shown in Fig. 4, after the first injection, resorption of the matrix portion of the implant occurs, leaving the non-resorbable component behind 400. The defect is thus reduced in size. For the second treatment, a smaller volume of the composition 402 would need to be injected into the remaining defect. After the matrix portion of the second injection resorbs, the defect is almost completely filled. A third injection may be needed, albeit a smaller volume than the first or second injection. With this approach, a soft tissue defect such as an acne scar or wrinkling may be filled gradually, with the added benefit of providing an implant that eventually provides a permanently fill to the defect in a cosmetically appealing manner. Similarly, a series of injections could also be performed into soft tissue such as the uvula, soft palate or pharyngeal wall. By doing so, the compliance of the tissue is gradually reduced, thereby preventing excessive tissue laxity that frequently results in obstructive sleep apnea.

In still yet another embodiment, the composition can be preformed into a device in a predetermined configuration prior to implantation. For example, simple molds such as trays, cylinders, and cups can be used to create parts such as films, rods, and hemispheres, respectively. The matrix and polymeric microparticles can then be injected into the mold and allowed to crosslink within the mold. The preformed device is then removed from the mold and is ready for sterilization and eventual implant into the tissue defect. In yet another embodiment of the invention, the predetermined configuration can be established on imaging data of the tissue defect or other sources. For example, a three dimensional computerized tomographic image of the defect is obtained, e.g., of an aortic aneurysm. After the image is obtained, it can be used to create a computer aided design (CAD) file. The CAD file is used to create a mold that mimics the tissue defect. The matrix and polymeric microparticles can then be injected and allowed to cure or crosslink within the mold. The preformed device is then removed from the mold and is ready for sterilization if necessary and eventual implantation into the tissue defect. In the case of a large tissue defect such as a disfiguring tumor of the head or neck, the pre-formed composition can be implanted directly into the space where the tumor was so that a normal appearance can be restored. Thus, a pre-formed "part" is obtained that can precisely be used to treat the tissue defect.

The following examples are given by way of illustration and not by way of limitation.

### EXAMPLE 1

A solution containing 100 mg of 35 micron diameter microparticles of polypropylene and 2 drops (50 mg) of surfactant Tween-60 was prepared. One thawed 5 ml vial of thrombin solution obtained from a Quixil™ kit (sold by Ethicon, Inc. of Somerville, NJ) was then added. The polypropylene microparticles are then easily dispersed throughout the thrombin solution. In earlier attempts to mix the polypropylene microparticles in thrombin solution, the particles did not disperse within the solution. Tween-60 helped to bring those particles into a dispersion. A thawed 5 ml vial of fibrinogen ("BAC") obtained from a Quixil™ kit was then added to the mixture. The total mixture was then allowed to rapidly form a rubbery solid white mass. This device could have been injected into a preformed mold and then implanted into a tissue defect. Alternatively, the device could have cured *in situ* by simultaneous delivery of the fibrinogen solution with the thrombin-polypropylene-Tween solution to a tissue defect by using a catheter, cannula or syringe.

### EXAMPLE 2

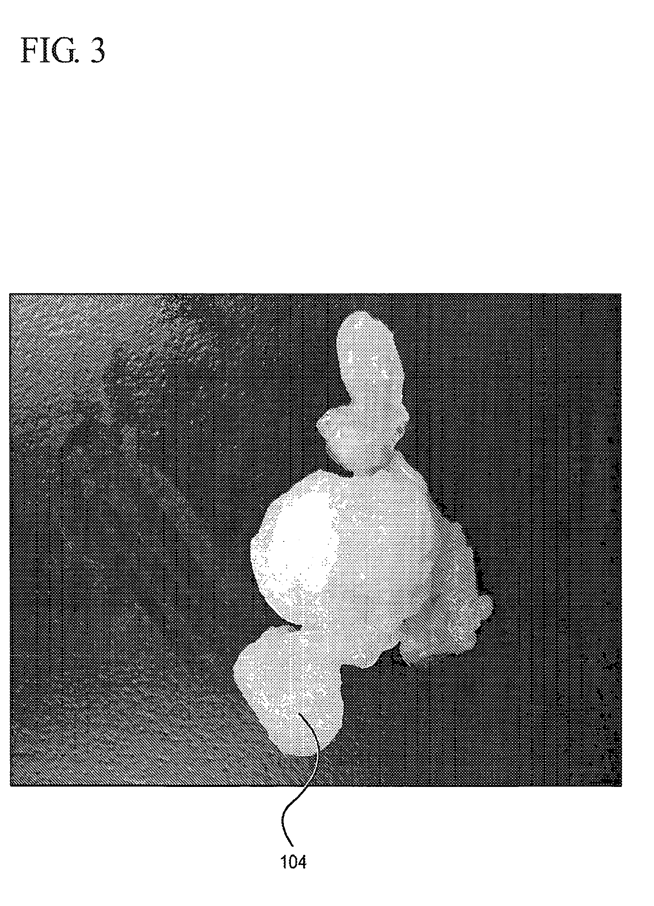

Fifty mg of gelatin powder and 50 mg polypropylene microparticles having an average diameter of 35 microns were added to a scintillation vial along with 50 mg of Tween-60. A 2 ml vial of thawed thrombin solution obtained from a Quixil™ kit was then added to mix the powders and form a uniform dispersion. The 2 ml vial of BAC - fibrinogen (also from a Quixil™ kit) was then added and the vial quickly agitated. Again, a rubbery gel was formed immediately. However, in this case, small domains of what appeared to be gelatin could be seen within the ball, equally disposed throughout the system, as shown in Fig. 3. The cured system had palpable turgidity. Addition of a gelatin component to the system may aid in crosslinking or solubilization of water soluble therapeutics, or modulating the turgidity of the gel. Similarly, a device comprised of hydroxylmethylpropyl cellulose, Tween 60, and polypropylene microparticles was also prepared. Accordingly, various amounts of gelatin powder or hydroxymethylcellulose can be used in the devices to achieve a variety of physicochemical properties.

### EXAMPLE 3

Two hundred mg of polypropylene microparticles having an average diameter of 35 microns were added to a scintillation vial along with 5 drops of Tween-60. Four ml of 1% w/v sodium alginate (in water) solution were then added to the vial. The mixture was vigorously agitated for 15 seconds until the microparticles were uniformly dispersed. Four ml of a 0.25 M CaCl₂ solution were then added to the mixture. A white opaque mass of uniformly dispersed microparticles was immediately obtained. The mass was removed from the vial and washed in distilled water to remove any free microparticles.

### EXAMPLE 4

A subcutaneous space of approximately 6 cm² was created just above the parietal peritoneum in a porcine abdomen. A 16G needle was then pushed into the abdominal wall until it could be seen pressing against the peritoneum. A 5 ml syringe containing 100 mg of 35 micron diameter polypropylene microparticles, 2 drops Tween-60, and 2 ml of freshly thawed thrombin solution obtained from a Quixil™ kit was then attached to the needle. The entire contents were injected into the supraperitoneal space. The syringe was removed and another syringe containing 2 ml of freshly thawed fibrinogen (also called the "biologic active component" or "BAC" component of the Quixil™ kit) solution was then coupled to the 16G needle. Quixil is supplied as two packages: a package containing BAC and Thrombin and an application device package. BAC and Thrombin are packaged together, in separate vials each containing 1 or 2 or 5 ml of frozen solution.

The entire contents were then injected into the supraperitoneal space so as to cause a reaction with the thrombin solution. The entire contents that were injected into the supraperitoneal space then cured. The injection site was then incised to observe that the two injections reacted. This was evident by the presence of a white compliant mass that had adhered to the tissue surrounding it.

### EXAMPLE 5

A subcutaneous space of approximately 10 cm² was created just above the parietal peritoneum in a porcine abdomen. A 16G needle was then pushed into the abdominal wall until it could be seen pressing against the peritoneum. A 5 ml syringe containing 250 mg of 35 micron diameter polypropylene microparticles, 5 drops Tween 60, and 5 ml of a 1% w/v sodium alginate solution was then attached to the needle. The entire contents were injected into the supraperitoneal space. The syringe was removed and another syringe containing 5 ml of 0.25 M CaCl₂ was then coupled to the 16G needle. The 5 ml of the 0.25 M CaCl₂ solution was then injected into the supraperitoneal space to cause the microparticles and sodium alginate to form a white compliant mass that had adhered to the tissue surrounding it. The microparticles were uniformly suspended within the matrix.

Unique advantages realized by the invention, particularly for percutaneous repair of hernia, include; less pain, less chance of infection, less dissection, reduced chance of seroma, improved compliance to tissue, eliminates localized stress and potential for recurrence at edge of mesh, stimulates long term ingrowth of tissue, easier to add drugs to modulate healing, less chance of nerve damage from needle sticks, no need to anchor to surrounding tissue, forms a plug, allows for self-dissection. The invention may also be useful in treating recurrent hernia. In effect, these compositions and devices can be implanted, molded, or injected to treat a variety of disease states involving cosmetic dermal defects, sphincter bulking, diverticulosis, GERD, aortic dissection, seroma, fallopian or vas deferens blockage, obstructive sleep apnea and embolics. In the case of obstructive sleep apnea, the composition can be injected into the uvula, soft palate, or pharyngeal walls to reduce the compliance of these tissues. The tissues are then less likely to collapse during sleep, thereby reducing the likelihood of obstructive sleep apnea.

Although the foregoing invention has been described in some detail by way of illustration and example for purposes of clarity and understanding, it will be obvious that certain changes and modifications may be practiced within the scope of the invention, as limited only by the scope of the appended claims.

## Claims

1. A kit suitable for treating tissue defects in mammals, the kit comprising:
first and second elements capable of polymerizing to form a resorbable polymer when combined, the first and second elements being thrombin and fibrinogen or sodium alginate and calcium chloride;
a surfactant; and
non-resorbable polymer microparticles;
wherein, in use, combining the first and second elements with the surfactant and microparticles forms a resorbable polymer matrix having the microparticles dispersed therein.

2. The kit according to claim 1, wherein the microparticles are dispersed within the matrix in a substantially uniform manner.

3. The kit according to claim 1 or 2, wherein the first and second elements are thrombin and fibrinogen.

4. The kit according to claim 1 or 2, wherein the first and second elements are sodium alginate and calcium chloride.

5. The kit according to claim 3, further comprising a fibrinolytic inhibitor.

6. The kit according to claim 3, further comprising micronized tantalum powder.

7. The kit according to claim 3, wherein the polymer microparticles are selected from the group consisting of polyethylene, polypropylene, polyurethane, silicone, and polytetrafluroethlyene.

8. The kit according to claim 3, wherein the polymer microparticles have a largest dimension of less than 1000 micrometers.

9. The kit according to claim 3, wherein the surfactant is selected from the group consisting of cationic, anionic and nonionic surfactants.

10. The kit according to claim 3, wherein the kit is further comprised of a therapeutic agent selected from the group consisting of anesthetics, anti-inflammatory agents, antimicrobial agents, antibiotics, or growth factors.

11. The kit according to claim 3, wherein the kit is further comprised of a tissue irritant selected from the group consisting of silk, talc, talcum powder, copper, metallic beryllium, wool, quartz dust, silica, crystalline silicates, poly(alkylcyanoacrylates), poly(ethylene-co-vinylacetate) chitosan, carbon tetrachloride, thioacetamide, fibrosin, ethanol, and bleomycin.

12. A method for preparing a composition suitable for treating tissue defects in mammals, comprising:
providing first and second elements capable of polymerizing to form a resorbable polymer when combined;
providing a surfactant;
providing non-resorbable polymer microparticles;
combining the first and second elements with the surfactant and microparticles to form a resorbable polymer matrix having the non-resorbable microparticles dispersed therein,
wherein the first and second elements are thrombin and fibrinogen, or sodium alginate and calcium chloride.

13. The method according to claim 12, wherein the first and second elements are thrombin and fibrinogen.

14. The method according to claim 12, wherein the first and second elements are sodium alginate and calcium chloride.

15. The method according to claim 12, wherein the tissue defect is selected from the group consisting of hernia, acne, wrinkles, emphysematous bullae, seroma, anal sphincter deficiency, and urinary sphincter deficiency.

## Patentansprüche

1. Für die Behandlung von Gewebedefekten bei Säugetieren geeignetes Kit, wobei das Kit Folgendes umfasst:
erste und zweite Elemente, die dazu fähig sind, unter Bildung eines resorbierbaren Polymers zu polymerisieren, wenn sie vereinigt werden, wobei es sich bei den ersten und den zweiten Elementen um Thrombin und Fibrinogen oder Natriumalginat und Calciumchlorid handelt;
ein Tensid und
nichtresorbierbare Polymermikropartikel;
wobei, bei der Anwendung, die ersten und die zweiten Elemente bei der Vereinigung mit dem Tensid und den Mikropartikeln eine resorbierbare Polymermatrix bilden, in der die Mikropartikel dispergiert sind.

2. Kit nach Anspruch 1, wobei die Mikropartikel in im Wesentlichen gleichmäßiger Weise in der Matrix dispergiert sind.

3. Kit nach Anspruch 1 oder 2, wobei es sich bei den ersten und den zweiten Elementen um Thrombin und Fibrinogen handelt.

4. Kit nach Anspruch 1 oder 2, wobei es sich bei den ersten und den zweiten Elementen um Natriumalginat und Calciumchlorid handelt.

5. Kit nach Anspruch 3, welches weiterhin einen fibrinolytischen Inhibitor umfasst.

6. Kit nach Anspruch 3, welches weiterhin mikronisiertes Tantalpulver umfasst.

7. Kit nach Anspruch 3, wobei die Polymermikropartikel aus der aus Polyethylen, Polypropylen, Polyurethan, Silikon und Polytetrafluorethylen bestehenden Gruppe ausgewählt sind.

8. Kit nach Anspruch 3, wobei die Polymermikropartikel eine größte Dimension von weniger als 1000 Mikrometer aufweisen.

9. Kit nach Anspruch 3, wobei das Tensid aus der aus kationischen, anionischen und nichtionischen Tensiden bestehenden Gruppe ausgewählt ist.

10. Kit nach Anspruch 3, wobei das Kit weiterhin ein aus der aus Anästhetika, entzündungshemmenden Mitteln, antimikrobiellen Mitteln, Antibiotika und Wachstumsfaktoren bestehenden Gruppe ausgewähltes therapeutisches Mittel umfasst.

11. Kit nach Anspruch 3, wobei das Kit weiterhin einen aus der aus Seide, Talkum, Talgpulver, Kupfer, metallischem Beryllium, Wolle, Quarzstaub, Siliziumdioxyd, kristallinen Silikaten, Poly(alkylcyanoacrylaten), Poly(ethylen-co-vinylacetat), Chitosan, Kohlenstofftetrachlorid, Thioacetamid, Fibrosin, Ethanol und Bleomycin bestehenden Gruppe ausgewählten gewebereizenden Stoff umfasst.

12. Verfahren zur Herstellung einer zur Behandlung von Gewebedefekten bei Säugetieren geeigneten Zusammensetzung, welches Folgendes umfasst:
die Bereitstellung von ersten und zweiten Elementen, die dazu fähig sind, unter Bildung eines resorbierbaren Polymers zu polymerisieren, wenn sie vereinigt werden;
die Bereitstellung eines Tensids;
die Bereitstellung von nichtresorbierbaren Polymermikropartikeln;
die Vereinigung der ersten und zweiten Elemente mit dem Tensid und den Mikropartikeln unter Bildung einer resorbierbaren Polymermatrix, in der die nichtresorbierbaren Mikropartikel dispergiert sind,
wobei es sich bei den ersten und zweiten Elementen um Thrombin und Fibrinogen oder Natriumalginat und Calciumchlorid handelt.

13. Verfahren nach Anspruch 12, wobei es sich bei den ersten und den zweiten Elementen um Thrombin und Fibrinogen handelt.

14. Verfahren nach Anspruch 12, wobei es sich bei den ersten und den zweiten Elementen um Natriumalginat und Calciumchlorid handelt.

15. Verfahren nach Anspruch 12, wobei der Gewebedefekt aus der aus Hernie, Akne, Falten, Emphysemblasen, Serom, einer Unzulänglichkeit des analen Schließmuskels und einer Unzulänglichkeit des Blasenschließmuskels bestehenden Gruppe ausgewählt ist.

## Revendications

1. Kit adapté au traitement des défauts tissulaires chez les mammifères, le kit comprenant :
un premier élément et un second élément pouvant polymériser pour former un polymère résorbable lorsqu'ils sont combinés, le premier élément et le second élément étant la thrombine et le fibrinogène ou l'alginate de sodium et le chlorure de calcium ;
un tensioactif ; et
des microparticules de polymère non résorbable ;
où, lors de l'utilisation, la combinaison du premier élément et du second élément avec le tensioactif et les microparticules forme une matrice polymère résorbable comportant les microparticules dispersées en son sein.

2. Kit conforme à la revendication 1, où les microparticules sont dispersées au sein de la matrice de façon substantiellement uniforme.

3. Kit conforme à la revendication 1 ou 2, où le premier élément et le second élément sont la thrombine et le fibrinogène.

4. Kit conforme à la revendication 1 ou 2, où le premier élément et le second élément sont l'alginate de sodium et le chlorure de calcium.

5. Kit conforme à la revendication 3, comprenant en outre un inhibiteur fibrinolytique.

6. Kit conforme à la revendication 3, comprenant en outre de la poudre de tantale micronisée.

7. Kit conforme à la revendication 3, où les microparticules de polymère sont choisies dans le groupe constitué par le polyéthylène, le polypropylène, le polyuréthane, la silicone et le polytétrafluoroéthylène.

8. Kit conforme à la revendication 3, où les microparticules de polymère présentent une plus grande dimension inférieure à 1000 micromètres.

9. Kit conforme à la revendication 3, où le tensioactif est choisi dans le groupe constitué par les tensioactifs cationiques, anioniques et non ioniques.

10. Kit conforme à la revendication 3, où le kit comprend en outre un agent thérapeutique choisi dans le groupe constitué par les anesthésiants, les agents anti-inflammatoires, les agents antimicrobiens, les antibiotiques et les facteurs de croissance.

11. Kit conforme à la revendication 3, où le kit comprend en outre un irritant tissulaire choisi dans le groupe constitué par les suivants : soie, talc, poudre de talc, cuivre, béryllium métallique, laine, poussière de quartz, silice, silicates cristallins, poly(alkylcyanoacrylates), poly(éthylène-co-acétate de vinyle), chitosane, tétrachlorure de carbone, thioacétamide, fibrosine, éthanol et bléomycine.

12. Procédé d'élaboration d'une composition adaptée au traitement des défauts tissulaires chez les mammifères, ledit procédé comprenant :
le fait de se munir d'un premier élément et d'un second élément pouvant polymériser pour former un polymère résorbable lorsqu'ils sont combinés ;
le fait de se munir d'un tensioactif ;
le fait de se munir de microparticules de polymère non résorbable ;
la combinaison du premier élément et du second élément avec le tensioactif et les microparticules pour former une matrice polymère résorbable comportant les microparticules dispersées en son sein,
le premier élément et le second élément étant la thrombine et le fibrinogène ou l'alginate de sodium et le chlorure de calcium.

13. Procédé conforme à la revendication 12, où le premier élément et le second élément sont la thrombine et le fibrinogène.

14. Procédé conforme à la revendication 12, où le premier élément et le second élément sont l'alginate de sodium et le chlorure de calcium.

15. Procédé conforme à la revendication 12, où le défaut tissulaire est choisi dans le groupe constitué par les suivants : hernie, acné, rides, emphysème bulleux, sérome, déficience du sphincter anal et déficience du sphincter urinaire.
